# EUROPEAN PATENT SPECIFICATION

(11) **EP 3 509 626 B1**
(45) Date of publication and mention of the grant of the patent: **13.10.2021**
(21) Application number: 17767784.6
(22) Date of filing: 08.09.2017
(51) Int. Cl.: C07K 14/78, C07K 14/82, A61K 38/39, A61K 38/00, A61K 39/395, C12N 9/12, A61K 31/506, A61K 45/06

(54) **FIBRONECTIN OR ILK INHIBITORS FOR USE IN THE TREATMENT OF LEUKEMIA**
FIBRONECTIN ZUR VERWENDUNG BEI DER BEHANDLUNG VON LEUKÄMIE
FIBRONECTINE À UTILISER DANS LE TRAITEMENT DE LA LEUCÉMIE

(30) Priority: 08.09.2016 EP 16187926
(43) Date of publication of application: 17.07.2019
(73) Proprietor: Chemotherapeutisches Forschungsinstitut Georg-Speyer-Haus, 60596 Frankfurt (DE)
(72) Inventor: KRAUSE, Daniela, 60598 Frankfurt am Main (DE); MEISTER, Melanie, 69469 Weinheim (DE); KUMAR, Rahul, 60596 Frankfurt am Main (DE)
(74) Representative: Kuttenkeuler, David
(86) International application number: PCT/EP2017/072591
(87) International publication number: WO 2018/046666

(56) References cited:
- US-B1- 6 177 273
- KASPAR M ET AL: "Fibronectin as target for tumor therapy", INTERNATIONAL JOURNAL OF CANCER, JOHN WILEY & SONS, INC, US, vol. 118, no. 6, 27 December 2005 (2005-12-27), pages 1331-1339, XP002396271, ISSN: 0020-7136, DOI: 10.1002/IJC.21677
- PILAR DE LA PUENTE ET AL: "Identification of ILK as a novel therapeutic target for acute and chronic myeloid leukemia", LEUKEMIA RESEARCH, vol. 39, no. 11, 1 November 2015 (2015-11-01), pages 1299-1308, XP055413815, US ISSN: 0145-2126, DOI: 10.1016/j.leukres.2015.09.005
- D. S. KRAUSE ET AL: "A hostel for the hostile: the bone marrow niche in hematologic neoplasms", HAEMATOLOGICA, THE HEMATOLOGY JOURNAL : OFFICIAL ORGAN OF THE EUROPEAN HEMATOLOGY ASSOCIATION, vol. 100, no. 11, 1 November 2015 (2015-11-01), pages 1376-1387, XP055332465, IT ISSN: 0390-6078, DOI: 10.3324/haematol.2014.113852
- ALWIN KRÄMER ET AL: "Adhesion to fibronectin stimulates proliferation of wild-type and bcr/abl-transfected murine hematopoietic cells", PROC. NATL ACAD SCI USA, vol. 96, no. 31, 1 March 1999 (1999-03-01) , pages 2087-2092, XP055332441,
- J. A. WERTHEIM: "BCR-ABL-induced adhesion defects are tyrosine kinase-independent", BLOOD, vol. 99, no. 11, 13 May 2002 (2002-05-13), pages 4122-4130, XP055332826, US ISSN: 0006-4971, DOI: 10.1182/blood.V99.11.4122
- BIN ZHANG ET AL: "Microenvironmental protection of CML stem and progenitor cells from tyrosine kinase inhibitors through N-cadherin and Wnt-b-catenin signaling", BLOOD, vol. 121, no. 10, 8 January 2013 (2013-01-08), pages 1824-1838, XP055332664, DOI: 10.1182/blood-2012-
- ADAM OBR ET AL: "Real-Time Analysis of Imatinib- and Dasatinib-Induced Effects on Chronic Myelogenous Leukemia Cell Interaction with Fibronectin", PLOS ONE, vol. 9, no. 9, 8 September 2014 (2014-09-08), page e107367, XP055332558, DOI: 10.1371/journal.pone.0107367
- J S DAMIANO ET AL: "Cell adhesion-mediated drug resistance (CAM-DR) protects the K562 chronic myelogenous leukemia cell line from apoptosis induced by BCR/ABL inhibition, cytotoxic drugs, and [gamma]-irradiation", LEUKEMIA., vol. 15, no. 8, 1 August 2001 (2001-08-01) , pages 1232-1239, XP055332483, US ISSN: 0887-6924, DOI: 10.1038/sj.leu.2402179

### SEQUENCE LISTING

<110> Chemotherapeutisches Forschungsinstitut Georg-Speyer-Haus
<120> FIBRONECTIN OR ILK INHIBITORS FOR USE IN THE TREATMENT OF LEUKEMIA
<130> U30739WO
<150> EP16187926.7
   <151> 2016-09-08
<160> 2
<170> PatentIn version 3.5
<210> 1
   <211> 452
   <212> PRT
   <213> Homo sapiens
<400> 1
   Met Asp Asp Ile Phe Thr Gln Cys Arg Glu Gly Asn Ala Val Ala Val
   1               5                   10                  15
   Arg Leu Trp Leu Asp Asn Thr Glu Asn Asp Leu Asn Gln Gly Asp Asp
               20                  25                  30
   His Gly Phe Ser Pro Leu His Trp Ala Cys Arg Glu Gly Arg Ser Ala
           35                  40                  45
   Val Val Glu Met Leu Ile Met Arg Gly Ala Arg Ile Asn Val Met Asn
       50                  55                  60
   Arg Gly Asp Asp Thr Pro Leu His Leu Ala Ala Ser His Gly His Arg
   65                  70                  75                  80
   Asp Ile Val Gln Lys Leu Leu Gln Tyr Lys Ala Asp Ile Asn Ala Val
                   85                  90                  95
   Asn Glu His Gly Asn Val Pro Leu His Tyr Ala Cys Phe Trp Gly Gln
               100                 105                 110
   Asp Gln Val Ala Glu Asp Leu Val Ala Asn Gly Ala Leu Val Ser Ile
           115                 120                 125
   Cys Asn Lys Tyr Gly Glu Met Pro Val Asp Lys Ala Lys Ala Pro Leu
       130                 135                 140
   Arg Glu Leu Leu Arg Glu Arg Ala Glu Lys Met Gly Gln Asn Leu Asn
   145                 150                 155                 160
   Arg Ile Pro Tyr Lys Asp Thr Phe Trp Lys Gly Thr Thr Arg Thr Arg
                   165                 170                 175
   Pro Arg Asn Gly Thr Leu Asn Lys His Ser Gly Ile Asp Phe Lys Gln
               180                 185                 190
   Leu Asn Phe Leu Thr Lys Leu Asn Glu Asn His Ser Gly Glu Leu Trp
           195                 200                 205
   Lys Gly Arg Trp Gln Gly Asn Asp Ile Val Val Lys Val Leu Lys Val
       210                 215                 220
   Arg Asp Trp Ser Thr Arg Lys Ser Arg Asp Phe Asn Glu Glu Cys Pro
   225                 230                 235                 240
   Arg Leu Arg Ile Phe Ser His Pro Asn Val Leu Pro Val Leu Gly Ala
                   245                 250                 255
   Cys Gln Ser Pro Pro Ala Pro His Pro Thr Leu Ile Thr His Trp Met
               260                 265                 270
   Pro Tyr Gly Ser Leu Tyr Asn Val Leu His Glu Gly Thr Asn Phe Val
           275                 280                 285
   Val Asp Gln Ser Gln Ala Val Lys Phe Ala Leu Asp Met Ala Arg Gly
       290                 295                 300
   Met Ala Phe Leu His Thr Leu Glu Pro Leu Ile Pro Arg His Ala Leu
   305                 310                 315                 320
   Asn Ser Arg Ser Val Met Ile Asp Glu Asp Met Thr Ala Arg Ile Ser
                   325                 330                 335
   Met Ala Asp Val Lys Phe Ser Phe Gln Cys Pro Gly Arg Met Tyr Ala
               340                 345                 350
   Pro Ala Trp Val Ala Pro Glu Ala Leu Gln Lys Lys Pro Glu Asp Thr
           355                 360                 365
   Asn Arg Arg Ser Ala Asp Met Trp Ser Phe Ala Val Leu Leu Trp Glu
       370                 375                 380
   Leu Val Thr Arg Glu Val Pro Phe Ala Asp Leu Ser Asn Met Glu Ile
   385                 390                 395                 400
   Gly Met Lys Val Ala Leu Glu Gly Leu Arg Pro Thr Ile Pro Pro Gly
                   405                 410                 415
   Ile Ser Pro His Val Cys Lys Leu Met Lys Ile Cys Met Asn Glu Asp
               420                 425                 430
   Pro Ala Lys Arg Pro Lys Phe Asp Met Ile Val Pro Ile Leu Glu Lys
           435                 440                 445
   Met Gln Asp Lys
       450
<210> 2
   <211> 1789
   <212> DNA
   <213> Homo sapiens
<400> 2
   gaattcatct gtcgactgct accacgggag ttccccggag aaggatcctg cagcccgagt      60
   cccgaggata aagcttgggg ttcatcctcc ttccctggat cactccacag tcctcaggct     120
   tccccaatcc aggggactcg gcgccgggac gctgctatgg acgacatttt cactcagtgc     180
   cgggagggca acgcagtcgc cgttcgcctg tggctggaca acacggagaa cgacctcaac     240
   cagggggacg atcatggctt ctcccccttg cactgggcct gccgagaggg ccgctctgct     300
   gtggttgaga tgttgatcat gcggggggca cggatcaatg taatgaaccg tggggatgac     360
   acccccctgc atctggcagc cagtcatgga caccgtgata ttgtacagaa gctattgcag     420
   tacaaggcag acatcaatgc agtgaatgaa cacgggaatg tgcccctgca ctatgcctgt     480
   ttttggggcc aagatcaagt ggcagaggac ctggtggcaa atggggccct tgtcagcatc     540
   tgtaacaagt atggagagat gcctgtggac aaagccaagg cacccctgag agagcttctc     600
   cgagagcggg cagagaagat gggccagaat ctcaaccgta ttccatacaa ggacacattc     660
   tggaagggga ccacccgcac tcggccccga aatggaaccc tgaacaaaca ctctggcatt     720
   gacttcaaac agcttaactt cctgacgaag ctcaacgaga atcactctgg agagctatgg     780
   aagggccgct ggcagggcaa tgacattgtc gtgaaggtgc tgaaggttcg agactggagt     840
   acaaggaaga gcagggactt caatgaagag tgtccccggc tcaggatttt ctcgcatcca     900
   aatgtgctcc cagtgctagg tgcctgccag tctccacctg ctcctcatcc tactctcatc     960
   acacactgga tgccgtatgg atccctctac aatgtactac atgaaggcac caatttcgtc    1020
   gtggaccaga gccaggctgt gaagtttgct ttggacatgg caaggggcat ggccttccta    1080
   cacacactag agcccctcat cccacgacat gcactcaata gccgtagtgt aatgattgat    1140
   gaggacatga ctgcccgaat tagcatggct gatgtcaagt tctctttcca atgtcctggt    1200
   cgcatgtatg cacctgcctg ggtagccccc gaagctctgc agaagaagcc tgaagacaca    1260
   aacagacgct cagcagacat gtggagtttt gcagtgcttc tgtgggaact ggtgacacgg    1320
   gaggtaccct ttgctgacct ctccaatatg gagattggaa tgaaggtggc attggaaggc    1380
   cttcggccta ccatcccacc aggtatttcc cctcatgtgt gtaagctcat gaagatctgc    1440
   atgaatgaag accctgcaaa gcgacccaaa tttgacatga ttgtgcctat ccttgagaag    1500
   atgcaggaca agtaggactg gaaggtcctt gcctgaactc cagaggtgtc gggacatggt    1560
   tgggggaatg cacctcccca aagcagcagg cctctggttg cctcccccgc ctccagtcat    1620
   ggtactaccc cagcctgggg tccatcccct tcccccatcc ctaccactgt gcgcaagagg    1680
   ggcgggctca gagctttgtc acttgccaca tggtgtcttc caacatggga gggatcagcc    1740
   ccgcctgtca caataaagtt tattatgaaa aaaaaaaaaa aaaaaaaaa                1789

## Description

The present invention pertains to fibronectin in the treatment of Chronic Myeloid Leukemia (CML) according to the appended set of claims . The invention relates to recombinant or isolated fibronectin for use as adjuvant therapy during Chronic Myeloid Leukemia (CML)treatment according to the appended set of claims, either as single ingredient medicament or in a combination therapy, preferably with ABL inhibitors such as imatinib or nilotinib.

### DESCRIPTION

Chronic Myeloid Leukemia (CML) is a hematological disorder which constitutes about 15% of adult leukemia, characterized by the malignant expansion of the myeloid lineage. The genetic hallmark of CML is a reciprocal translocation between chromosomes 9 and 22 resulting in the so- called Philadelphia (Ph) chromosome. The molecular consequence of this inter-chromosomal exchange is the creation of the chimeric gene BCR-ABL1, encoding a tyrosine kinase polypeptide in which the tyrosine kinase domain is constitutively activated. The expression of this fusion protein has been shown to be necessary and sufficient for the transformed phenotype of CML cells. In addition to CML, deregulated ABL kinase activity resulting from the Ph chromosomal translocation is also detected in up to 20% of adult lymphoblastic leukemia (ALL) patients.

Hematological cancers have a high frequency amongst the general population and pose a major health care problem. Significant progress has been made in the treatment of leukemia. The advent of the BCR-ABL1-targeting tyrosine kinase inhibitor (TKI) imatinib mesylate (IM) (marketed as Gleevec® or Glivec®) in 2001 increased the 5-year survival rate of patients to 90%. However, leukemic stem cells (LSC) are not eradicated (Corbin AS, Agarwal A, Loriaux M, Cortes J, Deininger MW, Druker BJ. Human chronic myeloid leukemia stem cells are insensitive to imatinib despite inhibition of BCR-ABL activity. J Clin Invest. 2011;121(1):396.) and 90% of patients relapse with mutations in BCR-ABL1 that lead to resistance towards IM (Shah NP, Nicoll JM, Nagar B, Gorre ME, Paquette RL, Kuriyan J, et al. Multiple BCR-ABL kinase domain mutations confer polyclonal resistance to the tyrosine kinase inhibitor imatinib (STI571) in chronic phase and blast crisis chronic myeloid leukemia. Cancer Cell. 2002;2(2): 117-25). Strikingly, most mutations leading to resistance towards IM occur in the Abl1 kinase domain, such as the point mutations of threonine 315 into isoleucine (BCR-ABL1T315I). The BCR-ABL1T315I mutation accounts for approximately 15% of all mutations found in patients during second line therapy, and for approximately 30% of mutations in patients on third line therapy (Soverini S, Branford S, Nicolini FE, Talpaz M, Deininger MW, Martinelli G, et al. Implications of BCR-ABL1 kinase domain-mediated resistance in chronic myeloid leukemia. Leuk Res. 2014;38(1): 10-20.). Interestingly, patients with the BCR-ABL1^{T315I} mutation have a rapid clinical course and poor prognosis, while for instance another mutation, the BCR-ABL1M351T mutation, is associated with slower clinical progression (Branford S, Rudzki Z, Walsh S, Parkinson I, Grigg A, Szer J, et al. Detection of BCR-ABL mutations in patients with CML treated with imatinib is virtually always accompanied by clinical resistance, and mutations in the ATP phosphate-binding loop (P-loop) are associated with a poor prognosis. Blood. 2003;102:276-83.). Second and third line TKIs such as nilotinib and dasatinib do not target the BCR-ABL1^{T315I} mutation and ponatinib, which is effective against the BCR-ABL1^{T315I} mutation, (and nilotinib and dasatinib) are frequently associated with severe adverse events (Moslehi JJ, Deininger M. Tyrosine Kinase Inhibitor-Associated Cardiovascular Toxicity in Chronic Myeloid Leukemia. J Clin Oncol. 2015;33(35):4210-8.), rendering the therapy for BCR-ABL1^{T315I}+ CML or B-cell acute lymphoblastic leukemia (B-ALL) extremely difficult and frequently unsuccessful. A novel strategy for targeting BCR-ABL1^{T315I}+ CML is needed.

The BMM or bone marrow niche represents the very complex arrangement of various cell types, all of which form the bone marrow niche for normal hematopoietic stem cells (HSC), the normal counterparts to LSC. Other factors determining the (patho-) physiology of the BMM are the oxygen tension, pH, mechanical forces, the extracellular matrix and cytokines. These various constituents affect the number, location, proliferation, self-renewal, and differentiation of hematopoietic stem and progenitor cells (HSPC) and likely similarly influence LSC. LSC interact with the BMM via specific pathways and are protected by the BMM to prevent chemotherapy- and TKI-induced apoptosis. As evidence of the BMM being a targetable entity in leukemia, it was previously demonstrated that modulation of the BMM by parathyroid hormone, the most potent regulator of bone turnover, led to a reduction of LSC in CML, but not AML, suggesting differential effects of the niche on myeloid leukemia (Krause DS, Fulzele K, Catic A, Sun CC, Dombkowski D, Hurley M, et al. Differential regulation of myeloid leukemia by the bone marrow microenvironment. Nat Med. 2013;19(11): 1513-7).

Integrin-linked kinase (ILK) is a protein Serine/Threonine kinase that binds to the cytoplasmic domains of β1, β2 and β3-integrin subunits. ILK serves as a molecular scaffold at sites of integrin-mediated adhesion, anchoring cytoskeletal actin and nucleating a supramolecular complex comprised minimally of ILK, PINCH and β-parvin. In addition to its structural role, ILK is a signaling kinase coordinating cues from the ECM in a phosphoinositide 3'-kinase (PI3K)-dependent manner following distinct signal inputs from integrins and growth factor receptor tyrosine kinases.

S. KRAUSE ET AL, "A hostel for the hostile: the bone marrow niche in hematologic neoplasms", HAEMATOLOGICA, THE HEMATOLOGY JOURNAL: OFFICIAL ORGAN OF THE EUROPEAN HEMATOLOGY ASSOCIATION, IT, (20151101), vol. 100, no. 11, review the emerging concepts of the normal and leukemic hematopoietic bone marrow niche and current models of how the microenvironment of the bone marrow may contribute to or be modified by leukemogenesis.

ALWIN KRAMER ET AL, "Adhesion to fibronectin stimulates proliferation of wild-type and bcr/abl-transfected murine hematopoietic cells", PROC. NATL ACAD SCI USA, (19990301), vol. 96, no. 31, pages 2087 - 2092, disclose that adhesion stimulates cell cycle progression of hematopoietic cells by downregulation of p27^{Klpl}, resulting in activation of cyclin A/CDK2 complexes and subsequent transition through the G1/S adhesion checkpoint.

J. A. WERTHEIM, "BCR-ABL-induced adhesion defects are tyrosine kinase-independent", BLOOD, US, (20020513), vol. 99, no. 11, doi:10.1182/blood.V99.11.4122, ISSN 0006-4971, pages 4122 - 4130, disclose that changes in adhesion induced by P210^{BCR-ABL} are independent of its tyrosine kinase activity.

BIN ZHANG ET AL, "Microenvironmental protection of CML stem and progenitor cells from tyrosine kinase inhibitors through N-cadherin and Wnt-b-catenin signaling", BLOOD, (20130108), vol. 121, no. 10, disclose an interplay mechanism between N-cadherin and the Wnt-β-catenin pathway in protecting CML LSCs during tyrosine kinase inhibitors (TKI) treatment.

It was therefore an object of the present invention to provide therapeutic options to provide novel treatments of leukemia. A further object of the invention was to overcome imatinib resistance during leukemia treatment.

The above problem is solved in a first aspect by fibronectin, or a functional variant thereof, as well as salts or solvates thereof, for use in the treatment or prevention of Chronic Myeloid Leukemia (CML).

The term "fibronectin", or "FN", as used in the present invention pertains to a protein expressed by a fibronectin gene as well as nucleic acid sequences encoding for the protein. The term shall comprise the various isoforms of fibronectin that occur through alternative splicing. Preferred are fibronectin proteins that are derived from the human fibronectin (*fn1*) gene locus. The *fn1* gene has the Genbank accession number 2335, and is furthermore annotated in other databases, such as in Ensembl:ENSG00000115414 HPRD:00626; MIM: 135600; Vega:OTTHUMG00000133054. The expression of its various splice forms is well known in the art and can be derived for example from Schwarzbauer et al 2011 ("Fibronectins, their fibrillogenesis, and in vivo functions."; Schwarzbauer JE1, DeSimone DW; Cold Spring Harb Perspect Biol. 2011 Jul 1;3(7). pii: a005041. doi: 10.1101/cshperspect.a005041). The fibronectin protein may be provided as a monomer or a dimer. Fibronectin is known to occur in a soluble form (formerly known as cold-insoluble globulin or CIg), which is a soluble FN dimer in which two FN monomers are covalently connected via disulfide bridges. This form occurs in the plasma and is a preferred form for use according to the present invention. However, FN protein is also present in higher order structures in an insoluble fibrillary form in the extracellular matrix.

In context of the invention the term fibronectin may both refer to the protein or the nucleic acid encoding such a protein. Therefore the invention in some embodiments also relates to a fibronectin encoding nucleic acid for use in the treatment or prevention of cancer. For example, the fibronectin encoding nucleic acid may occur in the form of an expression vector comprising a fibronectin encoding nucleic acid operably linked to a promoter sequence.

A fibronectin in context of the invention is in some embodiments preferably in the form of the isolated recombinant and/or purified protein in a composition, which means that the protein is in a non-naturally occurring composition prepared for the therapeutic administration to a mammal. Such a composition may be serum or plasma preparation, or a blood preparation with an increased concentration of soluble fibronectin. In some other embodiments the fibronectin composition of the invention is not a blood transfusion, plasma or serum preparation, but a composition wherein fibronectin is the only blood derived component. In some embodiments the fibronectin composition is a cryoprecipitate.

A proteinaceous fibronectin for use according to the present invention is either a recombinantly produced protein, or a protein purified from a blood or plasma sample from a donor. The donor may be selected from a healthy donor or from an autologous blood or plasma sample. The FN protein for use according to the invention is preferably a full length human fibronectin, comprising not more than 20 mutations, wherein a mutation is selected from an amino acid substitution, deletion, insertion or addition, compared to the sequence of any isoform of human fibronectin as known in the art.

The term "cancer" shall refer to any proliferative cancerous disorders, and preferably includes solid and liquid cancers. Most preferably a cancer of the invention is leukemia. The term "leukemia" refers broadly to progressive, malignant diseases of the blood-forming organs and is generally characterized by a distorted proliferation and development of leukocytes and their precursors in the blood and bone marrow. Leukemia is generally clinically classified on the basis of the duration and character of the disease-acute or chronic; the type of cell involved; myeloid (myelogenous), lymphoid, or monocytic; and the increase or non-increase in the number of abnormal cells in the blood-leukemic or aleukemic (subleukemic). Accordingly, the present invention includes fibronectin for use in treating leukemia, including treating acute myeloid leukemia, chronic lymphocytic leukemia, acute promyelocytic leukemia, adult T-cell leukemia, aleukemic leukemia, a leukocythemic leukemia, basophilic leukemia, blast cell leukemia, bovine leukemia, chronic myelocytic leukemia, leukemia cutis, embryonal leukemia, eosinophilic leukemia, Gross' leukemia, hairy-cell leukemia, hemoblastic leukemia, hemocytoblastic leukemia, histiocytic leukemia, stem cell leukemia, acute monocytic leukemia, leukopenic leukemia, lymphatic leukemia, lymphoblastic leukemia, lymphocytic leukemia, lymphogenous leukemia, lymphoid leukemia, lymphosarcoma cell leukemia, mast cell leukemia, megakaryocyte leukemia, micro myeloblastic leukemia, monocytic leukemia, myeloblastic leukemia, myelocytic leukemia, myeloid granulocytic leukemia, myelomonocytic leukemia, Naegeli leukemia, plasma cell leukemia, multiple myeloma, plasmacytic leukemia, promyelocytic leukemia, Rieder cell leukemia, Schilling's leukemia, stem cell leukemia, subleukemic leukemia, and undifferentiated cell leukemia.

Most preferably treated in context with the present invention is a Bcr-Abll positive leukemia, such as a Chronic Myelogenous Leukemia (CML) or Acute Lymphocytic Leukemia (ALL). Also it is preferable in context of the invention that the leukemia is an imatinib-resistant leukemia, preferably a Bcr-Abll mutant positive, preferably imatinib resistant, leukemia. A Bcr-Abll mutant in context of the invention is preferably selected from the group consisting of M351T, F359V, H396P, I432T, F486S, M244V, L248V, G250E, Y253H, Y253F, E255K, K263E, L273M, T315I, F317L, and N331S; and preferably is the Bcr-Abl^{T315I} mutant.

The treatment of leukemia according to the invention may furthermore comprise chemotherapy, radiation therapy or stem cell transplantation approaches. As used herein, the term "bone marrow transplantation" or "stem cell transplantation" or "hematopoietic stem cell transplantation" used herein should be considered as interchangeable, referring to the transplantation of stem cells in some form to a recipient. The terms shall include both allogenic as well as autologous stem cell transplantations. The stem cells do not necessarily have to be derived from bone marrow, but could also be derived from other sources such as umbilical cord blood.

Therefore, in some embodiments the fibronectin may be used in context of a stem cell or bone marrow transplantation for a Chronic Myeloid Leukemia (CML) therapy according to the invention, wherein the to be transplanted stem cells are contacted with fibronectin before transplantation, or wherein the fibronectin is administered concomitantly with the stem cells to the patient.

Preferably, the treatment according to the invention comprises the administration of a therapeutically effective amount of fibronectin, preferably to the bone marrow of a subject suffering from the Chronic Myeloid Leukemia (CML). The administration of fibronectin may be local, however, the administration is preferably systemic, for example by intravenous injection.

A systemic administration in context of the present invention refers to oral, rectal, and parenteral (i.e., intramuscular, intravenous, and subcutaneous) routes for administration. Generally, it will be found that when the compounds of the invention are administered orally a larger quantity of the active agent is required to produce the same effect as a smaller quantity given parenterally. In accordance with good clinical practice, it is preferred to administer the compounds at a concentration level that will produce effective therapeutic effect without causing any harmful or untoward side effects.

The "therapeutically effective dose" of the compounds of the invention in a composition for purposes herein is determined by such considerations as are known in the art. The dose must be effective to achieve improvement including but not limited to an improved course of disease, more rapid recovery, and improvement of symptoms, elimination of symptoms and other indicators as are selected as appropriate measures by those skilled in the art. The compounds of the invention can be administered in a single dose or in multiple doses.

In general, the active dose of compound for humans is in the range of from 1 ng/kg to about 20-100 mg/kg body weight per day, preferably about 0.01 mg/kg to about 2-10 mg/kg body weight per day, in a regimen of one dose per day or twice or three or more times per day for a single dose or multiple dose regimen.

In some embodiments it will be preferred that fibronectin is used as an adjuvant during a Chronic Myeloid Leukemia (CML) treatment. In these embodiments the treatment according to the invention comprises the concomitant or sequential use of fibronectin and at least one other anti-cancer agent. Alternatively, the treatment comprises the use of a combination of fibronectin or a functional variant thereof, as well as salts or solvates thereof, and at least one other anti-cancer agent.

An "Anti-cancer agent" in accordance with the invention shall refer to a composition (e.g. compound, drug, antagonist, inhibitor, modulator) having antineoplastic properties or the ability to inhibit the growth or proliferation of cells. In embodiments, an anticancer agent is a chemotherapeutic. In embodiments, an anti-cancer agent is an agent approved by the EMA, or FDA, or similar regulatory agency of a country other than the European Union, for treating cancer. Examples of anti-cancer agents include, but are not limited to, MEK (e.g. MEK1, MEK2, or MEK1 and MEK2) inhibitors (e.g. XL518, CI-1040, PD035901, selumetinib/ AZD6244, GSK1120212/ trametinib, GDC-0973, ARRY-162, ARRY-300, AZD8330, PD0325901, U0126, PD98059, TAK-733, PD318088, AS703026, BAY 869766), alkylating agents (e.g., cyclophosphamide, ifosfamide, chlorambucil, busulfan, melphalan, mechlorethamine, uramustine, thiotepa, nitrosoureas, nitrogen mustards (e.g., mechloroethamine, cyclophosphamide, chlorambucil, melphalan), ethylenimine and methylmelamines (e.g., hexamethlymelamine, thiotepa), alkyl sulfonates (e.g., busulfan), nitrosoureas (e.g., carmustine, lomustine, semustine, streptozocin), triazenes (decarbazine)), anti-metabolites (e.g., 5- azathioprine, leucovorin, capecitabine, fludarabine, gemcitabine, pemetrexed, raltitrexed, folic acid analog (e.g., methotrexate), or pyrimidine analogs (e.g., fluorouracil, floxouridine, Cytarabine), purine analogs (e.g., mercaptopurine, thioguanine, pentostatin), etc.), plant alkaloids (e.g., vincristine, vinblastine, vinorelbine, vindesine, podophyllotoxin, paclitaxel, docetaxel, etc.), topoisomerase inhibitors (e.g., irinotecan, topotecan, amsacrine, etoposide (VP 16), etoposide phosphate, teniposide, etc.), antitumor antibiotics (e.g., doxorubicin, adriamycin, daunorubicin, epirubicin, actinomycin, bleomycin, mitomycin, mitoxantrone, plicamycin, etc.), platinum-based compounds (e.g. cisplatin, oxaloplatin, carboplatin), anthracenedione (e.g., mitoxantrone), substituted urea (e.g., hydroxyurea), methyl hydrazine derivative (e.g., procarbazine), or adrenocortical suppressant (e.g., mitotane, aminoglutethimide), epipodophyllotoxins (e.g., etoposide).

Further examples of anti-cancer agents include, but are not limited to, antibiotics (e.g., daunorubicin, doxorubicin, bleomycin), enzymes (e.g., L-asparaginase), inhibitors of mitogen- activated protein kinase signaling (e.g. U0126, PD98059, PD184352, PD0325901, ARRY- 142886, SB239063, SP600125, BAY 43-9006, wortmannin, or LY294002), mTOR inhibitors, antibodies (e.g., rituxan), 5-aza-2'-deoxycytidine, doxorubicin, vincristine, etoposide, gemcitabine, imatinib (Gleevec.RTM.), geldanamycin, 17-N-Allylamino-17- Demethoxygeldanamycin (17-AAG), bortezomib, trastuzumab, anastrozole; angiogenesis inhibitors; antiandrogen, antiestrogen; antisense oligonucleotides; apoptosis gene modulators; apoptosis regulators; arginine deaminase; BCR/ABL1 antagonists; beta lactam derivatives; bFGF inhibitor; bicalutamide; camptothecin derivatives; casein kinase inhibitors (ICOS); clomifene analogues; cytarabine dacliximab; dexamethasone; estrogen agonists; estrogen antagonists; etanidazole; etoposide phosphate; exemestane; fadrozole; finasteride; fludarabine; fluorodaunorunicin hydrochloride; gadolinium texaphyrin; gallium nitrate; gelatinase inhibitors; gemcitabine; glutathione inhibitors; hepsulfam; immunostimulant peptides; insulin-like growth factor- 1 receptor inhibitor; interferon agonists; interferons; interleukins; letrozole; leukemia inhibiting factor; leukocyte alpha interferon; leuprolide+estrogen+progesterone; leuprorelin; matrilysin inhibitors; matrix metalloproteinase inhibitors; MIF inhibitor; mifepristone; mismatched double stranded RNA; monoclonal antibody,; mycobacterial cell wall extract; nitric oxide modulators; oxaliplatin; panomifene; pentrozole; phosphatase inhibitors; plasminogen activator inhibitor; platinum complex; platinum compounds; prednisone; proteasome inhibitors; protein A-based immune modulator; protein kinase C inhibitor; protein kinase C inhibitors, protein tyrosine phosphatase inhibitors; purine nucleoside phosphorylase inhibitors; ras farnesyl protein transferase inhibitors; ras inhibitors; ras-GAP inhibitor; ribozymes; signal transduction inhibitors; signal transduction modulators; single chain antigen-binding protein; stem cell inhibitor; stem-cell division inhibitors; stromelysin inhibitors; synthetic glycosaminoglycans; tamoxifen methiodide; telomerase inhibitors; thyroid stimulating hormone; translation inhibitors; tyrosine kinase inhibitors; urokinase receptor antagonists; steroids (e.g., dexamethasone), finasteride, aromatase inhibitors, gonadotropin-releasing hormone agonists (GnRH) such as goserelin or leuprolide, adrenocorticosteroids (e.g., prednisone), progestins (e.g.,hydroxyprogesterone caproate, megestrol acetate, medroxyprogesterone acetate), estrogens (e.g., diethlystilbestrol, ethinyl estradiol), antiestrogen (e.g., tamoxifen), androgens (e.g., testosterone propionate, fluoxymesterone), antiandrogen (e.g., flutamide), immunostimulants (e.g., Bacillus Calmette-Guerin (BCG), levamisole, interleukin-2, alpha- interferon, etc.), monoclonal antibodies (e.g., anti-CD20, anti-HER2, anti-CD52, anti-HLA-DR, and anti-VEGF monoclonal antibodies), immunotoxins (e.g., anti-CD33 monoclonal antibody-calicheamicin conjugate, anti-CD22 monoclonal antibody-pseudomonas exotoxin conjugate, etc.), radioimmunotherapy (e.g., anti- CD20 monoclonal antibody conjugated to U 1ln, 90Y, or 1311, etc.), triptolide, homoharringtonine, dactinomycin, doxorubicin, epirubicin, topotecan, itraconazole, vindesine, cerivastatin, vincristine, deoxyadenosine, sertraline, pitavastatin, irinotecan, clofazimine, 5-nonyloxytryp-tamine, vemurafenib, dabrafenib, erlotinib, gefitinib, EGFR inhibitors, epidermal growth factor receptor (EGFR)-targeted therapy or therapeutic (e.g. gefitinib (Iressa™), erlotinib (Tarceva™), cetuximab (Erbitux™), lapatinib (Tykerb™), panitumumab (Vectibix™), vandetanib (Caprelsa™), afatinib/BIBW2992, CI-1033/canertinib, neratinib/HKI-272, CP-724714, TAK-285, AST-1306, ARRY334543, ARRY-380, AG-1478, dacomitinib/PF299804, OSI-420/desmethyl erlotinib, AZD8931, AEE788, pelitinib/EKB-569, CUDC-101, WZ8040, WZ4002, WZ3146, AG-490, XL647, PD153035, BMS-599626), sorafenib, imatinib, sunitinib, nilotinib, dasatinib, or the like.

In another preferred embodiment the anti-cancer agent in this aspect may be a compound which is selected from an anti-leukemic agent, and preferably is an ILK-inhibitor as mentioned below, nilotinib , dasatinib, ponatinib or imatinib or the allosteric Abl001 inhibitor. The allosteric ABL001 inhibitor and its derivatives is disclosed in Wylie AA et al., "The allosteric inhibitor ABL001 enables dual targeting of BCR-ABL1". Nature. 2017 Mar 30;543(7647):733-737, in particular reference is made to the structural information therein regarding the ABL001 inhibitor.

In some preferred embodiments of the invention the least one anti-cancer agent is a kinase inhibitor, preferably a tyrosine kinase inhibitor such as an inhibitor of Platelet-derived growth factor receptor (PDGFR), c-Abl and/or Src kinase inhibitors.

Yet further preferable is that the tyrosine kinase inhibitor for use in the combinatorial treatments of the invention is imatinib or nilotinib, and/or salts thereof, such as the methanesulfonate salt of imatinib (also known as Gleevec®). Imatinib mesylate is chemically 4-[(4-Methyl 1-piperazinyl)methyl]-N-[4- methyl-3-[[4-(3-pyridinyl)-2-pyrimidinyl]amino]-phenyl]benzamide methanesulfonate and is used to treat chronic myelogenous leukemia (CML), gastrointestinal stromal tumors (GISTs) and other cancers.

The term "nilotinib" as used herein includes nilotinib in the form of free base, a pharmaceutically acceptable salt thereof, amorphous nilotinib, crystalline nilotinib or any isomer, hydrate, solvate, or prodrug or combinations thereof. "Salts" or "pharmaceutically acceptable salt(s)", as used herein, include but are not limited to inorganic or organic salts, hydrates and solvates of nilotinib known to persons skilled in the art.

The subject to be treated with the fibronectin in accordance with the present invention is preferably a subject suffering from Chronic Myeloid Leukemia (CML). Preferably such a subject is a naive patient who did not receive any treatment. Alternatively preferred is that the subject in need of the treatment of the invention is a subject that already underwent a treatment, such as a treatment with a tyrosine kinase inhibitor, e.g. imatinib. In some embodiments the subject is a refractory subject, hence, a subject who underwent a treatment with for example imatinib, and the treatment was successful but after the treatment experienced a relapse. Most preferably the subject to be treated in context of the invention is a patient who suffers from an imatinib resistant form of leukemia.

In a further aspect of the present invention here is provided a combination comprising (a) fibronectin, or a functional variant thereof, as well as salts or solvates thereof, and (b) Bcr-Abl1-inhibitor, the latter being preferably selected from imatinib and nilotinib as defined herein above, for use in the treatment of Chronic Myeloid Leukemia (CML).

Yet another aspect of the invention then pertains to a pharmaceutical composition comprising fibronectin, or a functional variant thereof, as well as salts or solvates thereof, and a pharmaceutically acceptable carrier, stabilizer and/or excipient for use in the treatment of Chronic Myeloid Leukemia (CML).

Pharmaceutical compositions comprising the fibronectin compounds of the invention are administered to a subject in need thereof by any number of routes including, but not limited to, topical, oral, intravenous, intramuscular, intra-arterial, intramedullary, intrathecal, intraventricular, transdermal, subcutaneous, intraperitoneal, intranasal, enteral, topical, sublingual, or rectal means.

In accordance with one embodiment, a method of treating a subject in need of such treatment is provided. The method comprises administering a pharmaceutical composition comprising a fibronectin of the present invention to a subject in need thereof.

The pharmaceutical compositions useful for practicing the invention may be administered to deliver a dose of between 1 ng/kg/day and 100 mg/kg/day.

The formulations of the pharmaceutical compositions described herein may be prepared by any method known or hereafter developed in the art of pharmacology. In general, such preparatory methods include the step of bringing the active ingredient into association with a carrier or one or more other accessory ingredients, and then, if necessary or desirable, shaping or packaging the product into a desired single- or multi-dose unit.

It will be understood by the skilled artisan that such pharmaceutical compositions are generally suitable for administration to animals of all sorts. Subjects to which administration of the pharmaceutical compositions of the invention is contemplated include, but are not limited to, humans and other primates, mammals including commercially relevant mammals such as cattle, pigs, horses, sheep, cats, and dogs, birds including commercially relevant birds such as chickens, ducks, geese, and turkeys. The invention is also contemplated for use in contraception for nuisance animals such as rodents.

A pharmaceutical composition of the invention may be prepared, packaged, or sold in bulk, as a single unit dose, or as a plurality of single unit doses. As used herein, a "unit dose" is a discrete amount of the pharmaceutical composition comprising a predetermined amount of the active ingredient. The amount of the active ingredient is generally equal to the dosage of the active ingredient which would be administered to a subject or a convenient fraction of such a dosage such as, for example, one-half or one-third of such a dosage.

The relative amounts of the active ingredient, the pharmaceutically acceptable carrier, and any additional ingredients in a pharmaceutical composition of the invention will vary, depending upon the identity, size, and condition of the subject treated and further depending upon the route by which the composition is to be administered. By way of example, the composition may comprise between 0.1% and 100% (w/w) active ingredient.

In addition to the active ingredient, a pharmaceutical composition of the invention may further comprise one or more additional pharmaceutically active agents, preferably anti-cancer agent.

Controlled- or sustained-release formulations of a pharmaceutical composition of the invention may be made using conventional technology.

As used herein, "additional ingredients" include, but are not limited to, one or more of the following: excipients; surface active agents; dispersing agents; inert diluents; granulating and disintegrating agents; binding agents; lubricating agents; sweetening agents; flavoring agents; coloring agents; preservatives; physiologically degradable compositions such as gelatin; aqueous vehicles and solvents; oily vehicles and solvents; suspending agents; dispersing or wetting agents; emulsifying agents, demulcents; buffers; salts; thickening agents; fillers; emulsifying agents; antioxidants; antibiotics; antifungal agents; stabilizing agents; and pharmaceutically acceptable polymeric or hydrophobic materials. Other "additional ingredients" which may be included in the pharmaceutical compositions of the invention are known in the art and described, for example in Genaro, ed., 1985, Remington's Pharmaceutical Sciences, Mack Publishing Co., Easton, Pa.,. Preferable is that the additional agents are suitable for the formulation of proteinaceous active ingredients.

Typically, dosages of the compound of the invention which may be administered to an animal, preferably a human, range in amount from 1 µg to about 100 g per kilogram of body weight of the animal. While the precise dosage administered will vary depending upon any number of factors, including but not limited to, the type of animal and type of disease state being treated, the age of the animal and the route of administration. In one aspect, the dosage of the compound will vary from about 1 mg to about 10 g per kilogram of body weight of the animal. In another aspect, the dosage will vary from about 10 mg to about 1 g per kilogram of body weight of the animal.

The compound may be administered to an animal as frequently as several times daily, or it may be administered less frequently, such as once a day, once a week, once every two weeks, once a month, or even less frequently, such as once every several months or even once a year or less. The frequency of the dose will be readily apparent to the skilled artisan and will depend upon any number of factors, such as, but not limited to, the type of cancer being diagnosed, the type and severity of the condition or disease being treated, the type and age of the animal, etc.

Suitable preparations include injectables, either as liquid solutions or suspensions, however, solid forms suitable for solution in, suspension in, liquid prior to injection, may also be prepared. The preparation may also be emulsified, or the polypeptides encapsulated in liposomes. The active ingredients are often mixed with excipients which are pharmaceutically acceptable and compatible with the active ingredient. Suitable excipients are, for example, water saline, dextrose, glycerol, ethanol, or the like and combinations thereof. In addition, if desired, the vaccine preparation may also include minor amounts of auxiliary substances such as wetting or emulsifying agents, pH buffering agents, and/or adjuvants.

The term "ILK" or "Integrin-linked Kinase" refers to a protein, mRNA or gene listed under the Human Gene Name ID: HGNC:6040 in the version of 1^{st} September 2017. Encompassed by the wording are also in some embodiments ILK homologs in mouse and rat. However preferred is the human version of ILK having a sequence that is at least 80% identical to the amino acid sequence shown in SEQ ID NO: 1, and preferably is 90%, most preferably 95 and most preferably 100% identical to the sequence shown in SEQ ID NO: 1. The mRNA sequence of human ILK is also shown herein as SEQ ID NO: 2.

The compound that is such a ILK inhibitor in context of the disclosure is selected from a polypeptide, peptide, glycoprotein, a peptidomimetic, an antigen binding construct (for example, an antibody, antibody-like molecule or other antigen binding deriva-tive, or an or antigen binding fragment thereof), a nucleic acid such as a DNA or RNA, for example an antisense or inhibitory DNA or RNA, a ribozyme, an RNA or DNA aptamer, RNAi, siRNA, shRNA and the like, including variants or derivatives thereof such as a peptide nucleic acid (PNA), a genetic construct for targeted gene editing, such as a CRISPR/Cas9 construct and/or a guide nucleic acid (gRNA or gDNA) and/or tracrRNA.

The compound may be an antigen binding construct such as an antibody or antibody-like molecule, or an antigen binding fragment thereof, which binds ILK. Preferably, the compound is an ILK inhibitory antibody, or an inhibitory antigen binding fragment thereof. Antibodies and inhibitory antibodies directed at ILK are well known in the art.

The compound maybe a nucleic acid such as an anti-sense nucleotide molecule such as a siRNA or shRNA molecule that binds to a nucleic acid that encodes ILK or regulates expression of ILK. Preferably the nucleic acid compound comprises a sequence that is complementary to or binds to, or is identical to, an mRNA encoding an ILK, such as preferably the nucleic acid shown in SEQ ID NO: 2, or a nucleic acid at least 80% identical thereto. Antisense inhibitors of ILK are for example described in U.S. Pat. No. 6,177,273.

The compound for use may be a molecule selected from Cpd22 (Lee, Su-Lin et al. "Identification and Characterization of a Novel Integrin-Linked Kinase Inhibitor." Journal of medicinal chemistry 54.18 (2011): 6364-6374.), QLT0267 (Kalra, Jessica et al. "QLT0267, a Small Molecule Inhibitor Targeting Integrin-Linked Kinase (ILK), and Docetaxel Can Combine to Produce Synergistic Interactions Linked to Enhanced Cytotoxicity, Reductions in P-AKT Levels, Altered F-Actin Architecture and Improved Treatment Outcomes in an Orthotopic Breast Cancer Model." Breast Cancer Research: BCR 11.3 (2009): R25. PMC. Web. 6 Sept. 2017), KP-392 (Liu J, Costello PC, Pham NA, Pintillie M, Jabali M, Sanghera J, Tsao MS, Johnston MR; J Thorac Oncol. 2006 Oct;1(8):771-9), KPSD1 or T315, or a derivative, isomer, salt, or solvate thereof. The art already contains many ILK inhibitory small molecules. The compound T315, or OSU-T315, is disclosed in Lee SL, Hsu EC, Chou CC, et al. Identification and characterization of a novel integrin-linked kinase inhibitor. J Med Chem. 2011;54(18):6364-6374. Other inhibitors of ILK can be found in U.S. Pat. No. 6,214,813.

The subject to be treated according to the invention is preferably a mouse, rat, guinea pig, rabbit, cat, dog, monkey, or preferably a human, for example a human patient.

In general, the treatment comprises a step of administering a therapeutically effective amount of the compound to the subject.

The present invention will now be further described in the following examples with reference to the accompanying figures and sequences, nevertheless, without being limited thereto. In the Figures:
- **Figure 1:**: Measurement of the shortest three-dimensional distance of a) normal (black) or BCR-ABL1+ (gray) lin- c-Kit+ Sca-1+ (LKS) or LKS CD150+ CD48-(LKS SLAM) cells, b) vehicle (black)- or imatinib (gray)-treated BCR-ABL1+ LKS cells and c) BCR-ABL1WT+ (black) versus BCR-ABL1T315I+ (gray) LKS cells labeled with the lipophilic dye DiD and injected into irradiated Col2.3kbGFP mice to the endosteum. The horizontal black line represents the mean.
- **Figure 2:**: Adhesion of sorted Mac-1+ BCR-ABL1T315I-positive cells from mice with CML to a) fibronectin or b) a murine stroma cell line.
- **Figure 3:**: Kaplan-Meier-survival curve of Balb/c recipient mice that were transplanted with BCR-ABL1WT (orange), BCR-ABL1Y253F (green), BCR-ABL1E255K (red), BCR-ABL1T315I (purple) and BCR-ABL1M351T (yellow) transduced bone marrow.
- **Figure 4:**: Southern blot from DNA isolated from mouse spleen from either control mice, BCR-ABL1T315IWT or BCR-ABL1T315I mice using a probe to GFP (a) and the quantification of clones per lane (b).
- **Figure 5:**: Immunofluorescence for fibronectin of 3T3 fibroblasts transduced with retroviruses expressing BCR-ABL1WT (left) or BCR-ABL1T315I (right).
- **Figure 6:**: Immunohistochemistry for fibronectin on bone sections of mice transplanted with empty-vector (left)-, BCR-ABL1 (middle) or BCR-ABL1T315I (right)-transduced cells taken at the time the mice were moribund.
- **Figure 7:**: White blood cell counts per µl in peripheral blood (a) and survival (b) of mice intravenously transplanted (no FN) or intrafemorally co-transplanted with BCR-ABL1 - or BCR-ABL1T315I -transduced bone marrow and vehicle or FN.
- **Figure 8:**: Kaplan-Meier survival curve of mice intravenously transplanted with BCR-ABL1 or BCR-ABL1T315I -transduced bone marrow treated with vehicle or FN on days 9, 10 and 12 after transplantation.
- **Figure 9:**: Kaplan-Meier-style survival curve for wildtype (solid line) or fibronectin (FN) fl x Colla2-Cre+ (dashed line) recipients of BCR-ABL1+ (black) or BCR-ABL1T315I+ (gray) bone marrow in the retroviral transduction/ transplantation model of chronic myeloid leukaemia (CML). While the BCR-ABL1T315I+ CML is not as aggressive as usual, there may be a trend towards disease acceleration in fibronectin (FN) fl x Colla2-Cre+ line recipients of BCR-ABL1+ bone marrow (black).

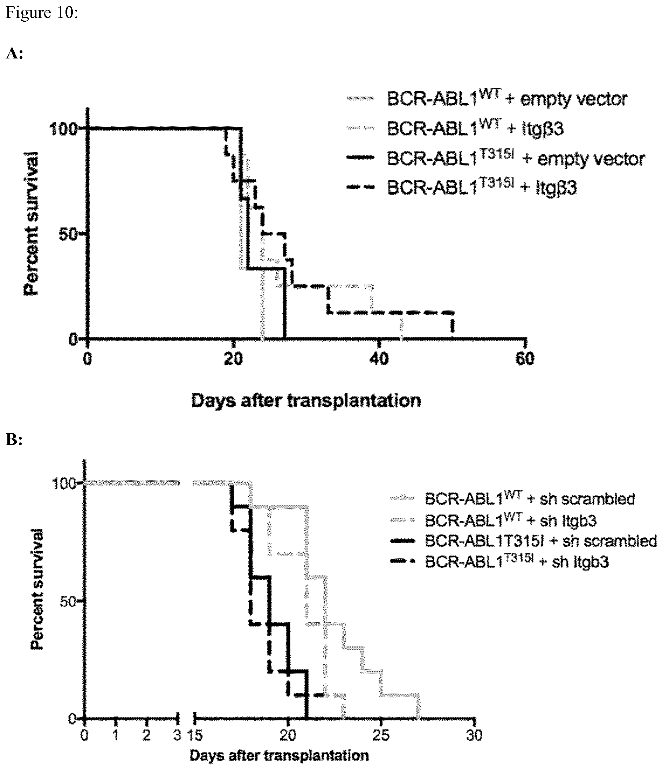

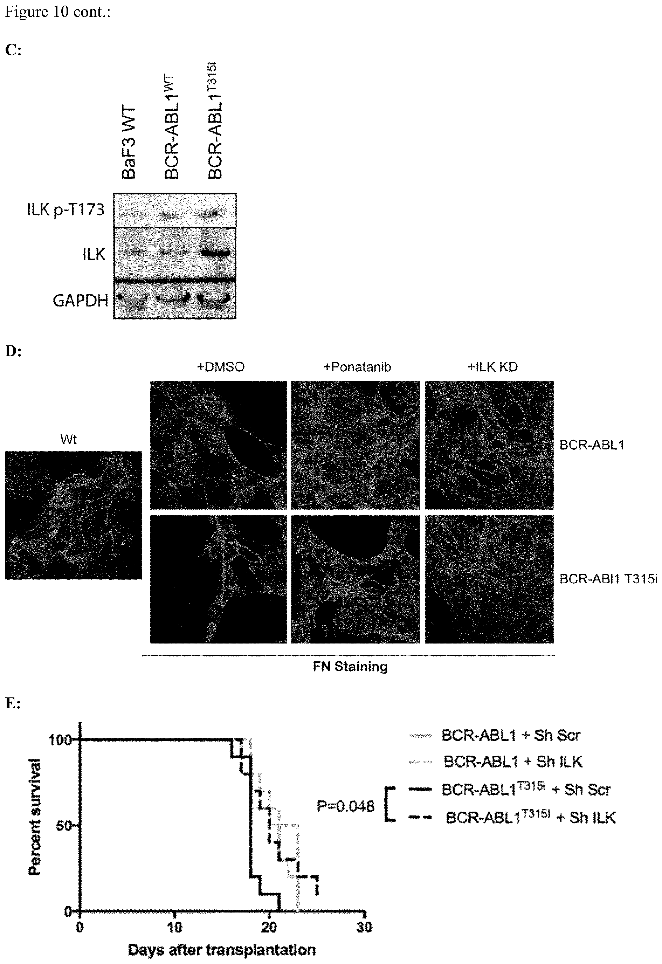

- **Figure 10:**: A: Kaplan-Meier-style survival curve for wildtype Balb/c recipients of BCR-ABL1+ (gray) or BCR-ABL1^{T315I}+ (black) bone marrow, cotransduced with empty vector (solid line)- or integrin β3 (dashed line)- overexpressing retrovirus in the retroviral transduction/ transplantation model of chronic myeloid leukaemia (CML). Possible differences are not statistically significant; B: Kaplan-Meier-style survival curve for wildtype Balb/c recipients of BCR-ABL1+ (gray) or BCR-ABL1^{T315I}+ (black) bone marrow, cotransduced with sh scrambled (solid line)- or sh integrin β3 (Itgb3) (dashed line)- expressing lentivirus in the retroviral transduction/ transplantation model of chronic myeloid leukaemia (CML). Possible differences are not statistically significant; C: Protein expression of integrin linked kinase (ILK) and pILK pT173 in lysates of wildtype BaF3 cells (BaF3 WT) or BaF3 cells transduced with BCR-ABL1WT or BCR-ABL1^{T315I}; D: Immunofluorescent staining for fibronectin deposition in wildtype (WT) 3T3 cells or 3T3 cells transduced with BCR-ABL1WT (top row) or BCR-ABL1^{T315I} (bottom row). Fibronectin staining is shown after treatment with vehicle (DMSO) (left), after treatment with the tyrosine kinase inhibitor ponatinib (middle) and after knockdown of integrin-linked kinase (ILK) (right). Treatment with ponatinib and knockdown of ILK restores fibronectin deposition in 3T3 cells transduced with BCR-ABL1^{T315I}; E: Kaplan-Meier-style survival curve for wildtype Balb/c recipients of BCR-ABL1+ (gray) or BCR-ABL1^{T315I}+ (black) bone marrow, cotransduced with sh scrambled (solid line)- or sh integrin-linked kinase (ILK) (dashed line)-expressing lentivirus in the retroviral transduction/ transplantation model of chronic myeloid leukaemia (CML) (*P* = 0.048).

### SEQ ID NO: 1 shows the sequence of human ILK:

### SEQ ID NO: 2 shows the mRNA sequence of human ILK:

### EXAMPLES

### Example 1: Hematopoietic stem cells and leukemia-initiating cells occupy distinct niches in the BMM

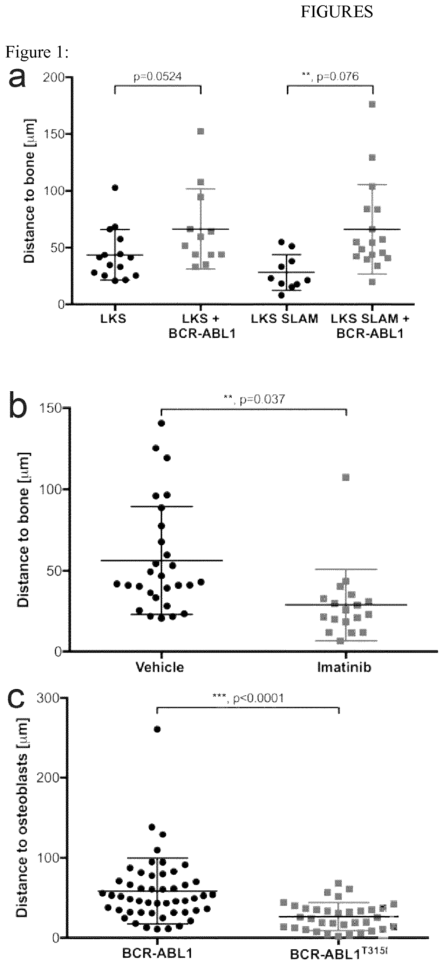

Using confocal 2-photon microscopy of the murine calvarium (skull) (15) and the murine retroviral transduction/transplantation model of CML induced by BCR-ABL1 or BCR-ABL1^{T315I} (16), the inventors could show that a) BCR-ABL1^{WT}-positive LSC home to locations further away from bone than normal HSC (Fig. 1 a), b) that imatinib-treatment reverses this phenotype and leads to closer localization of BCR- ABL1WT+ LSC to the endosteum than treatment with vehicle (Fig. 1 b) and c) that LSC positive for BCR- ABL1^{T315I} homed significantly closer to the bone than BCR-ABL1^{WT} LSC (Fig. 1 c).

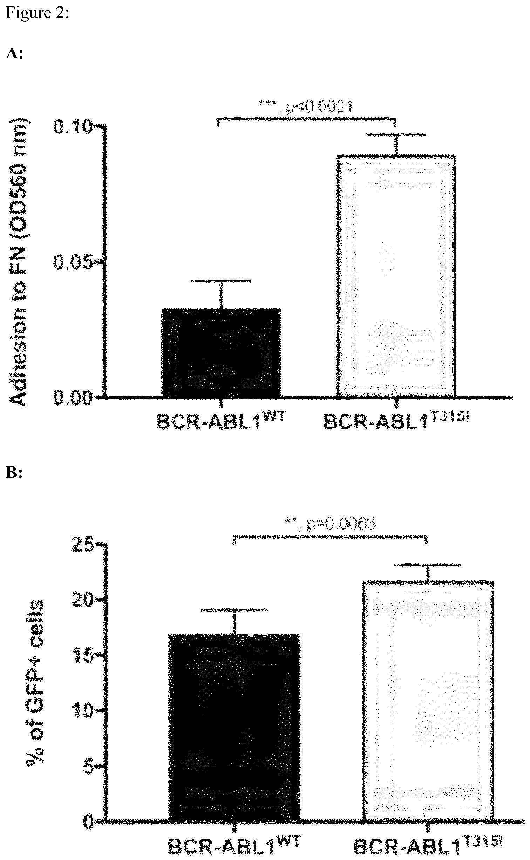

Furthermore, increased adhesion of BCR-ABL1^{T315I}+ compared to BCR-ABL1WT+ myeloid cells to fibronectin (Fig. 2 a) and stroma (Fig. 2 b) was demonstrated by an adhesion assay, respectively, suggesting that an altered interaction with the BMM may be associated with altered clinical outcome.

### Example 2: CML survival depends on the mutation status in the BCR-ABL1 kinase

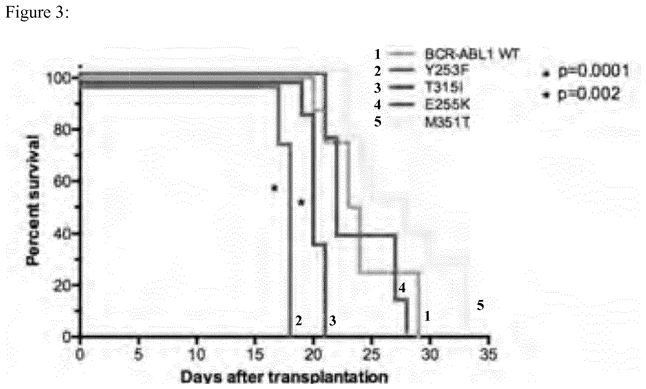

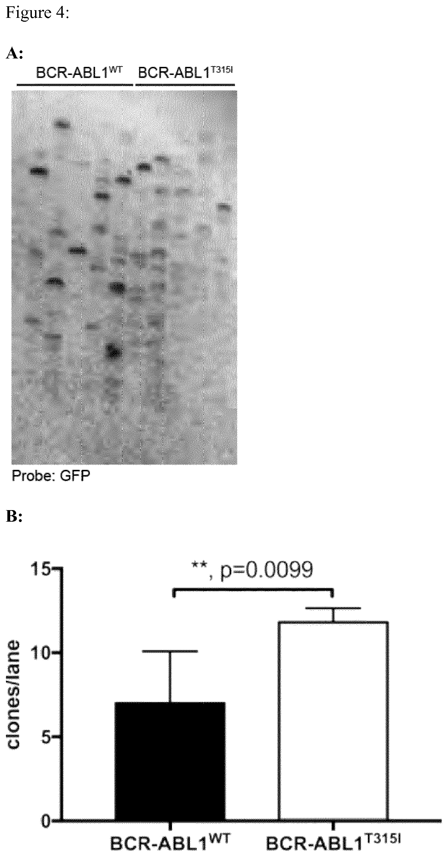

In the same murine model recipients of bone marrow transduced with BCR-ABL1^{WT} died by day 30, while recipients of BCR-ABL1^{M351T}-transduced bone marrow had prolonged and recipients of BCR-ABL1^{T315I} or BCR-ABL1^{Y253F}-transduced bone marrow had shortened survival (Fig. 3), exactly recapitulating the survival in human patients (1). Consistently, measurement of the engraftment of BCR-ABL1+ clones by Southern blotting (9, 14) revealed an increase in clonality of the BCR-ABL1^{T315I}+ compared to the BCR-ABL1^{WT}+ disease (Fig. 4a and 4h; P<0.01), suggesting that the increased engraftability likely correlates with clinical course (9, 14) and possibly localization in the niche.

### Example 3: Interaction with fibronectin influences the aggressivity of CML

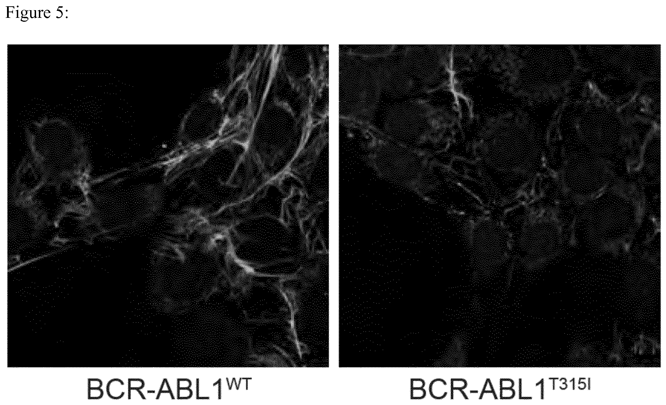

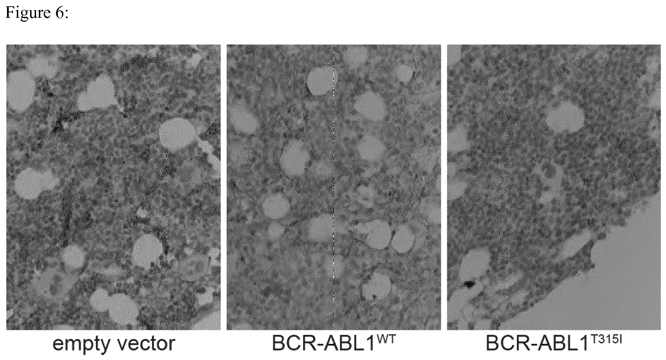

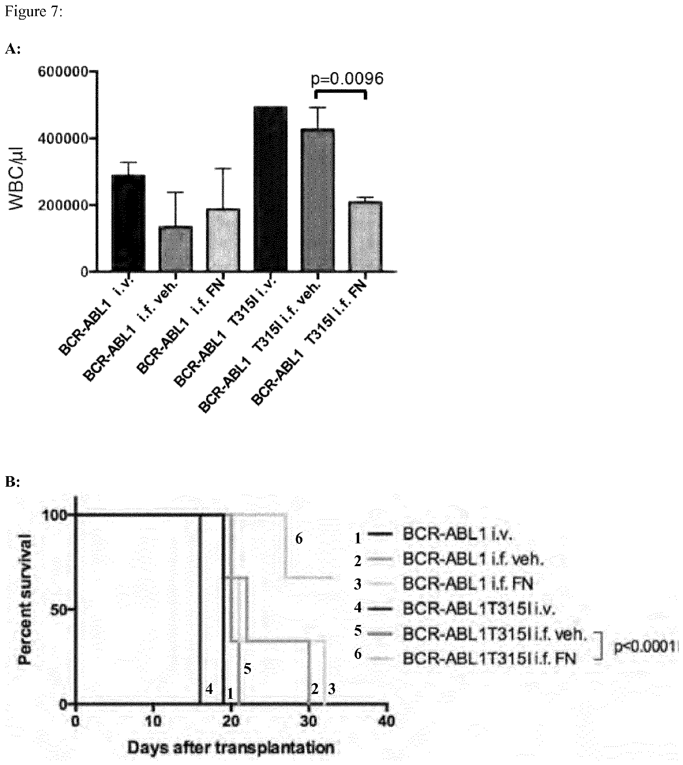

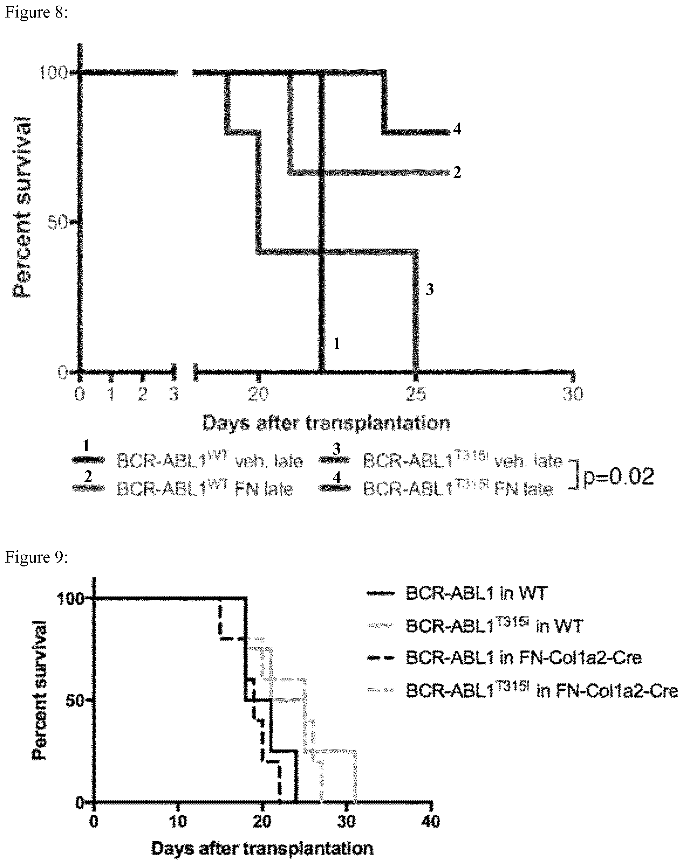

The inventors have shown by immunofluorescence (Fig. 5) and immunohistochemistry of bone sections of mice transplanted with empty vector-transduced cells, or mice with BCR-ABL1^{WT}+ or BCR-ABL1^{T315I}+ CML-like myeloproliferative neoplasia (Fig. 6), that BCR-ABL1^{T315I}+ cells deposit less fibronectin in their environment than BCR-ABL1^{WT}+ cells, which is likely also reflected by their differences in their interaction with the BMM. Importantly, intrafemoral co-injection of fibronectin and BCR-ABL1^{WT}+ or BCR-ABL1^{T315I}+ leukemia-initiating cells significantly decreased the tumor burden (Fig. 7a) and prolonged survival in recipients of BCR-ABL1^{T315I}-transduced bone marrow compared to recipients of the same bone marrow but intrafemorally co-injected with vehicle (Fig. 7b). In a more translational experiment it was further demonstrated that three intravenous injections of fibronectin on days 9, 10 and 12 post transplantation led to a significant prolongation of survival in BCR-ABL1^{T315I}+ CML compared to vehicle-treated mice with this disease (Fig. 8).

Taken together, the data suggest that substitution of (pathologically decreased) fibronectin in the BMM of mice with BCR-ABL1^{T315I}+ CML leads to reduced aggressivity and prolonged survival in this extremely aggressive form of leukemia.

Depending on the prior treatment regimen the BCR-ABL1^{T315I} mutation occurs in approximately 15-30% of all CML patients in whom mutations conveying resistance to TKIs have been found. In addition, the BCR-ABL1^{T315I} mutation can be found in blastic phase CML and in B-ALL. The presence of this mutation is frequently associated with worse outcome and treatment options are limited, especially in view of the frequent side effects of ponatinib. Novel treatments, ideally those with a different mode of attack, are urgently needed.

Intravenous injection or transfusion of fibronectin according to the herein described invention is a feasible novel form of treatment for BCR-ABL1^{T315I}+ CML and possibly B-ALL. Fibronectin is present in plasma and enriched in cryoprecipitate, a form of plasma, which was frozen, thawed to a slush stage, centrifuged and frozen. Fibronectin could, therefore, be obtained from healthy blood donors or may be produced recombinantly.

### Example 4: Lack of fibronectin in the bone marrow microenvironment accelerates BCR-ABL1^{WT+} CML

In order to test, whether deficiency of fibronectin in the leukemic environment may accelerate BCR-ABL1⁺ CML, the inventors induced BCR-ABL1+ or BCR-ABL1^{T315I}+ CML in mice with inducible fibroblast-specific knockout of fibronectin (fibronectin fl x Colla2-Cre-ER) or in wildtype mice. Indeed, this led to a trend towards acceleration of disease in fibronectin fl x Colla2-Cre-ER mice transplanted with BCR-ABL1⁺ bone marrow (Fig. 9).

### Example 5: Mechanism of altered fibronectin deposition in BCR-ABL1^{T315I+} CML - the role of integrin β3 and integrin linked kinase

The question was how the fibronectin-integrin-signaling pathway differs between BCR-ABL1⁺ and BCR-ABL1^{T315I}+ CML and, therefore why BCR-ABL1^{T315I}+ CML is specifically sensitive to treatment with fibronectin. BCR-ABL1^{T315I}+ CML express higher levels of integrin β3 than BCR-ABL1⁺ cells, but lower levels of fibronectin. Overexpression of integrin β3 on BCR-ABL1^{T315I}+ CML cells led to a trend towards prolonged survival (Fig. 10A), while, complementarily, knockdown of integrin β3 led to a trend towards accelerated disease progression in BCR-ABL1^{T315I}+ CML (Fig. 10A). Integrin-linked kinase (ILK), which is a scaffold protein involved in focal adhesion points, the signal transduction of β-integrins and the deposition of fibronectin, was found to be more phosphorylated in BCR-ABL1^{T315I}+ CML compared to BCR-ABL1⁺ CML (Fig. 10B). Treatment of BCR-ABL1⁺ versus BCR-ABL1^{T315I+} cells with ponatinib, a tyrosine kinase inhibitor effective against the BCR-ABL1^{T315I+} mutation, or knockdown of ILK led to an increase in the deposition of fibronectin (Fig. 10C), suggesting that a) fibronectin deposition via ILK is a BCR-ABL1 kinase-dependent process and that b) ILK is involved in the reduced deposition of fibronectin in BCR-ABL1^{T315I}+ CML. Consequently, knockdown of ILK in BCR-ABL1^{T315I}+ donor bone marrow significantly prolonged survival (Fig. 10D) showing that inhibition of ILK is beneficial in BCR-ABL1^{T315I}+ CML

### Materials and Methods

### Antibodies and Reagents

Immunohistochemistry: Fibronectin (Abcam, ab2413, Cambridge, UK); Immunofluorescence: AlexaFluor-647 goat anti-rabbit (Molecular Probes/Thermo Fisher, Waltham, USA), fibronectin (Abcam, ab2413, Cambridge, UK).

Murine fibronectin was purchased from Abcam (ab92784, Cambridge, UK), lyophilized bovine fibronectin was purchased from Thermo Fisher (33010018, Waltham, USA, prepared as 1 mg/ml in HBSS).

### Mice

BALB/c mice were purchased from Charles River Laboratories (Sulzfeld, Germany). All animal studies were approved by the government in the German region of Hessen (Regierungspräsidium Darmstadt).

### Bone marrow transduction and transplantation

Our retroviral stock was generated and bone marrow transplantation experiments were performed as described earlier (9, 14, 16). In general, we injected between 2.25 and 2.5 x 105 transduced cells into female irradiated (750 cGy) Balb/c recipient mice. The T315I point mutation in BCR-ABL1 was introduced by site-directed mutagenesis in the open reading frame of the MSCV IRES GFP vector.

### Treatment of mice

Therapeutic application of fibronectin was performed as follows: intravenous application of 200 µg bovine fibronectin (in HBSS) on days 9, 10 and 12 post transplantation; for vehicle control treatments, 200 µl of HBSS (equal volume to fibronectin dose) was administered. For intrafemoral injections, 50 µg murine fibronectin was injected on days 0, 1 and 2 post transplantation; for vehicle control treatments, 50 µl HBSS was used.

### Fibronectin Adhesion Assay

Adhesion to fibronectin was tested as described by the manufacturer (Cell Biolabs Inc., San Diego, USA). In brief, 150.000 cells per fibronectin-coated well of a 24-well-plate were allowed to adhere for 90 min. After vigorous washing with PBS cells were stained with the supplied staining solution and after washing with water extracted with the supplied extraction solution. Wells coated with bovine serum albumin (BSA) were used as control. Adhesion was measured in a spectrophotometer at OD560.

### Immunofluorescence

Transduced 3T3 fibroblasts were allowed to adhere and to grow on round coverslips of a 15 mm diameter for at least 24 hours. Alternatively, non-adherent BaF3 or primary bone marrow cells were cytospun onto polysine-coated slides (Menzel Gläser. Braunschweig). Cells were either fixed in pure methanol for 10 min at -20°C or in 4% paraformaldehyde (PFA) (Morphisto, Franfurt am Main) for 10 min at room temperature. PFA-fixed cells were permeabilised with 0.25% Triton in PBS for 5 min. Prior to incubation with primary antibodies cells were blocked in 2% BSA in PBS for 10 min at room temperature. Primary antibodies were generally diluted 1:100 in 2% BSA/PBS and cells were incubated for 1 h at room temperature. After 2 washing steps in PBS for 5 min each, the cells were incubated for 1 h at room temperature with fluorophore-labeled secondary antibodies (diluted 1:300), including a counterstain for nuclei with 5 µg/ml DAPI (Merck, Darmstadt). Cells were washed in PBS and briefly in water and mounted with FluoroMount plus 50 µg/ml 1,4-diazabicyclo(2,2,2)octan (DABCO) (Sigma-Aldrich, Munich). Specimen were analyzed using a confocal laser scanning microscope (Leica, SP5, Wetzlar) and pictures were managed and analyzed with ImageJ.

### Histology/Immunohistochemistry

Bones were immersed in formalin for at least 24 h. Consequently, bones were decalcified with 0.5 M EDTA for 5 days with an exchange of EDTA after the first 24 h. Bones were then kept in formalin until mounting in paraffin. Bones were sectioned, de-paraffinised and either stained with hematoxylin & eosin for histopathological analysis or with an antibody directed to fibronectin (Abcam, ab2413, Cambridge UK) following standard procedures.

### Southern Blotting

DNA was extracted using phenol/chloroform and precipitated with isopropanol. The DNA was then digested with the restriction endonuclease BglII, separated by agarose gel electrophoresis and transferred to a nylon membrane. Subsequently, DNA was hybridized with a radioactively labeled probe derived from the GFP gene to detect proviral integration sites, as described (14).

### Statistical analysis

All statistical analyses were performed using GraphPad Prism software. Statistical tests such as unpaired, two-tailed Student's t-test, one- or two-way ANOVA tests were used where appropriate. In general, p-values below 0.05 were considered significant (p<0.05, *). Survival curves were analysed with Kaplan-Meier-style survival curves and log-rank (Mantel-Cox) or Gehan-Breslow-Wilcoxon tests.

### References:

1. Branford S, Rudzki Z, Walsh S, Parkinson I, Grigg A, Szer J, et al. Detection of BCR-ABL mutations in patients with CML treated with imatinib is virtually always accompanied by clinical resistance, and mutations in the ATP phosphate-binding loop (P-loop) are associated with a poor prognosis. Blood. 2003;102:276-83.
2. Corbin AS, Agarwal A, Loriaux M, Cortes J, Deininger MW, Druker BJ. Human chronic myeloid leukemia stem cells are insensitive to imatinib despite inhibition of BCR-ABL activity. J Clin Invest. 2011;121(1):396.
3. Shah NP, Nicoll JM, Nagar B, Gorre ME, Paquette RL, Kuriyan J, et al. Multiple BCR-ABL kinase domain mutations confer polyclonal resistance to the tyrosine kinase inhibitor imatinib (STI571) in chronic phase and blast crisis chronic myeloid leukemia. Cancer Cell. 2002;2(2): 117-25.
4. Soverini S, Branford S, Nicolini FE, Talpaz M, Deininger MW, Martinelli G, et al. Implications of BCR-ABL1kinase domain-mediated resistance in chronic myeloid leukemia. Leuk Res. 2014;38(1): 10-20.
5. Moslehi JJ, Deininger M. Tyrosine Kinase Inhibitor-Associated Cardiovascular Toxicity in Chronic Myeloid Leukemia. J Clin Oncol. 2015;33(35):4210-8.
6. Krause DS, Scadden DT. A hostel for the hostile: the bone marrow niche in hematologic neoplasms. Haematologica. 2015;100(11): 1376-87.
7. Lundell BI, Mc Carthy JB, Kovach NL, Verfaillie CM. Activation of beta1 integrins on CML progenitors reveals cooperation between beta1 integrins and CD44 in the regulation of adhesion and proliferation. Leukemia. 1997;11:822-9.
8. Tanaka R, Owaki T, Kamiya S, Matsunaga T, Shimoda K, Kodama H, et al. VLA-5-mediated adhesion to fibronectin accelerates hemin-stimulated erythroid differentiation of K562 cells through induction of VLA-4 expression. J Biol Chem. 2009;284(30): 19817-25.
9. Krause DS, Lazarides K, von Andrian UH, Van Etten RA. Requirement for CD44 in homing and engraftment of BCR-ABL-expressing leukemic stem cells. Nat Med. 2006;12(10): 1175-80.
10. Krause DS, Lazarides K, Lewis JB, von Andrian UH, Van Etten RA. Selectins and their ligands are required for homing and engraftment of BCR-ABL1+ leukemic stem cells in the bone marrow niche. Blood. 2014;123(9): 1361-71.
11. Jin L, Hope KJ, Zhai Q, Smadja-Joffe F, Dick JE. Targeting of CD44 eradicates human acute myeloid leukemic stem cells. Nat Med. 2006;12(10): 1167.
12. Ishikawa F, Yoshida S, Saito Y, Hijikata A, Kitamura H, Tanaka S, et al. Chemotherapy-resistant human AML stem cells home to and engraft within the bone-marrow endosteal region. Nat Biotechnol. 2007;25(11):1315-21.
13. Zhang B, Li M, McDonald T, Holyoake TL, Moon RT, Campana D, et al. Microenvironmental protection of CML stem and progenitor cells from tyrosine kinase inhibitors through N-cadherin and Wnt-beta-catenin signaling. Blood. 2013;121 (10):1824-38.
14. Krause DS, Fulzele K, Catic A, Sun CC, Dombkowski D, Hurley M, et al. Differential regulation of myeloid leukemias by the bone marrow microenvironment. Nat Med. 2013;19(11): 1513-7.
15. Lo Celso C, Fleming HE, Wu JW, Zhao CX, Miake-Lye S, Fujisaki J, et al. Live-animal tracking of individual haematopoietic stem cells in their niche. Nature. 2009;2009(457):92-6.
16. Li S, Ilaria RL, Million RP, Daley GQ, Van Etten RA. The P190, P210, and P230 forms of the BCR/ABL oncogene induce a similar chronic myeloid leukemia-like syndrome in mice but have different lymphoid leukemogenic activity. J Exp Med. 1999;189(9):1399-412.

## Claims

1. Fibronectin, or a functional variant thereof, as well as salts or solvates thereof, for use in the treatment or prevention of Chronic Myeloid Leukemia (CML).

2. The fibronectin for use according to claim 1, wherein the CML is Bcr-Abl1 positive.

3. The fibronectin for use according to any one of claims 1 to 2, wherein the cancer is an imatinib-resistant leukemia, preferably a Bcr-Abl1 mutant positive leukemia.

4. The fibronectin for use according to claim 3, wherein the Bcr-Abl1 mutant is selected from the group consisting of M351T, F359V, H396P, I432T, F486S, M244V, L248V, G250E, Y253H, Y253F, E255K, K263E, L273M, T315I, F317L, and N331S; and preferably is Bcr-Abl1^{T315I}.

5. The fibronectin for use according to any one of claims 1 to 4, wherein the fibronectin or a functional variant thereof, is either a recombinantly produced protein, or a protein purified from a blood sample from a healthy donor.

6. The fibronectin for use according to any one of the preceding claims, wherein the treatment comprises bone marrow transplantation.

7. The fibronectin for use according to any one of the preceding claims, wherein the treatment comprises the concomitant or sequential use of fibronectin and at least one other anti-cancer agent.

8. The fibronectin for use according to any one of the preceding claims, wherein the treatment comprises the use of a combination of fibronectin or a functional variant thereof, as well as salts or solvates thereof, and at least one other anti-cancer agent.

9. The fibronectin for use according to claim 7 or 8, wherein at least one anti-cancer agent is a kinase inhibitor, preferably a tyrosine kinase such as a Bcr-Abl kinase and/or Src kinase inhibitor, preferably imatinib or nilotinib or dasatinib or ponatinib or the allosteric ABL inhibitor ABL001, or salts thereof.

10. The fibronectin for use according to any one of the preceding claims, wherein the fibronectin, or a functional variant thereof, as well as salts or solvates thereof, is administered to a patient suffering from leukemia subsequent or concomitantly to a bone marrow transplantation.

11. A combination for use in the treatment of CML, the combination comprising (a) fibronectin, or a functional variant thereof, as well as salts or solvates thereof, and (b) Abl1 -inhibitor or an inhibitor of the expression, function and/or stability of integrin-linked kinase (ILK).

12. The combination for use according to claim 11, wherein the inhibitor of the expression, function and/or stability of integrin-linked kinase (ILK) is (i) an ILK inhibitory antibody, or an inhibitory antigen binding fragment thereof, or (ii) an anti-sense nucleotide molecule that binds to a nucleic acid that encodes ILK, or (iii) is a molecule selected from Cpd22, QLT0267, KP-392 or T315, or an isomer, salt, or solvate thereof.

13. A pharmaceutical composition for use in the treatment of CML, comprising fibronectin, or a functional variant thereof, as well as salts or solvates thereof, and a pharmaceutically acceptable carrier, stabilizer and/or excipient.

14. The combination for use according to any one of claims 11 to 12, wherein the CML is a Bcr-Abli positive CML.

15. The combination for use according to any one of claims 11, 12 or 14, wherein the CML is an imatinib-resistant CML, preferably a Bcr-Abli mutant positive CML, such as Bcr-Abl1^{T315I} positive CML.

## Patentansprüche

1. Fibronektin, oder eine funktionelle Variante davon, sowie Salze oder Solvate davon zur Verwendung bei der Behandlung oder Vorbeugung von chronischer myeloischer Leukämie (CML).

2. Fibronektin zur Verwendung nach Anspruch 1 oder 2, wobei die CML Bcr-Abl1 ist.

3. Fibronektin zur Verwendung nach einem der Ansprüche 1 bis 2, wobei der Krebs eine Imatinib-resistente Leukämie, vorzugsweise eine Bcr-Abl1-Mutanten-positive Leukämie ist.

4. Fibronektin zur Verwendung nach Anspruch 3, wobei die Bcr-Abl1-Mutante aus der Gruppe ausgewählt ist, die aus M351T, F359V, H396P, I432T, F486S, M244V, L248V, G250E, Y253H, Y253F, E255K, K263E, L273M, T315I, F317L und N331S besteht; und vorzugsweise Bcr-Abl1^{T315I} ist.

5. Fibronektin zur Verwendung nach einem der Ansprüche 1 bis 4, wobei das Fibronektin, oder eine funktionelle Variante davon, entweder ein rekombinant hergestelltes Protein oder ein aus einer Blutprobe eines gesunden Spenders aufgereinigtes Protein ist.

6. Fibronektin zur Verwendung nach einem der vorhergehenden Ansprüche, wobei die Behandlung eine Knochenmarktransplantation umfasst.

7. Fibronektin zur Verwendung nach einem der vorhergehenden Ansprüche, wobei die Behandlung die gleichzeitige oder aufeinanderfolgende Verwendung von Fibronektin und wenigstens einem anderen Antikrebsmittel umfasst.

8. Fibronektin zur Verwendung nach einem der vorhergehenden Ansprüche, wobei die Behandlung die Verwendung einer Kombination von Fibronektin, oder einer funktionellen Variante davon, sowie Salzen oder Solvaten davon und wenigstens einem anderen Antikrebsmittel umfasst.

9. Fibronektin zur Verwendung nach Anspruch 7 oder 8, wobei wenigstens ein Antikrebsmittel ein Kinasehemmer, vorzugsweise ein Tyrosinkinasehemmer wie etwa ein Bcr-Abl-Kinase- und/oder Src-Kinasehemmer, vorzugsweise Imatinib oder Nilotinib oder Dasatinib oder Ponatinib oder der allosterische ABL-Hemmer ABL001 oder Salze davon ist.

10. Fibronektin zur Verwendung nach einem der vorhergehenden Ansprüche, wobei das Fibronektin, oder eine funktionelle Variante davon, sowie Salze oder Solvate davon einem an Leukämie leidenden Patienten nach oder gleichzeitig mit einer Knochenmarktransplantation verabreicht wird.

11. Kombination zur Verwendung bei der Behandlung von CML, wobei die Kombination Folgendes umfasst: (a) Fibronektin, oder eine funktionelle Variante davon, sowie Salze oder Solvate davon und (b) Abl1-Hemmer oder einen Hemmer der Expression, Funktion und/oder Stabilität von Integrin-verknüpfter Kinase (integrin-linked kinase - ILK).

12. Kombination zur Verwendung nach Anspruch 11, wobei der Hemmer der Expression, Funktion und/oder Stabilität von Integrin-verknüpfter Kinase (ILK) (i) ein ILKhemmender Antikörper oder ein hemmendes Antigen-bindendes Fragment davon ist, oder (ii) ein Antisense-Nukleotidmolekül, das an eine Nukleinsäure bindet, die für ILK kodiert, oder (iii) ein Molekül ist, das aus Cpd22, QLT0267, KP-392 oder T315, oder einem Isomer, Salz oder Solvat davon ausgewählt ist.

13. Pharmazeutische Zusammensetzung zur Verwendung bei der Behandlung von CML, die Fibronektin, oder eine funktionelle Variante davon, sowie Salze oder Solvate davon und einen pharmazeutisch verträglichen Träger, Stabilisator und/oder Hilfsstoff umfasst.

14. Kombination zur Verwendung nach einem der Ansprüche 11 bis 12, wobei die CML eine Bcr-Abl1-positive CML ist.

15. Kombination zur Verwendung nach einem der Ansprüche 11, 12 oder 14, wobei die CML eine Imatinib-resistente CML, vorzugsweise eine Bcr-Abl1-Mutanten-positive CML, wie etwa Bcr-Abl1-^{T315I}-positive CML, ist.

## Revendications

1. Fibronectine, ou un variant fonctionnel de celle-ci, ainsi que ses sels ou solvates, à utiliser dans le traitement ou la prévention de la leucémie myéloïde chronique (LMC).

2. Fibronectine à utiliser selon la revendication 1 ou 2, la LMC étant Bcr-Abl1.

3. Fibronectine à utiliser selon l'une quelconque des revendications 1 à 2, le cancer étant une leucémie résistante à l'imatinib, de préférence une leucémie positive mutante à Bcr-Abl1.

4. Fibronectine à utiliser selon la revendication 3, le mutant Bcr-Abl1 étant choisi dans le groupe constitué par M351T, F359V, H396P, I432T, F486S, M244V, L248V, G250E, Y253H, Y253F, E255K, K263E, L273M, T315I, F317L et N331S ; et étant de préférence Bcr-Abl1^{T315I}.

5. Fibronectine à utiliser selon l'une quelconque des revendications 1 à 4, la fibronectine ou un variant fonctionnel de celle-ci étant soit une protéine produite par recombinaison, soit une protéine purifiée à partir d'un échantillon de sang provenant d'un donneur en bonne santé.

6. Fibronectine à utiliser selon l'une quelconque des revendications précédentes, le traitement comprenant une transplantation de moelle osseuse.

7. Fibronectine à utiliser selon l'une quelconque des revendications précédentes, le traitement comprenant l'utilisation concomitante ou séquentielle de la fibronectine et d'au moins un autre agent anticancéreux.

8. Fibronectine à utiliser selon l'une quelconque des revendications précédentes, le traitement comprenant l'utilisation d'une combinaison de fibronectine ou d'un variant fonctionnel de celle-ci, ainsi que de ses sels ou solvates, et d'au moins un autre agent anticancéreux.

9. Fibronectine à utiliser selon la revendication 7 ou 8, au moins un agent anticancéreux étant un inhibiteur de kinase, de préférence un inhibiteur de tyrosine kinase tel qu'un inhibiteur de kinase Bcr-Abl et/ou Src, de préférence l'imatinib ou le nilotinib ou le dasatinib ou le ponatinib ou l'inhibiteur ABL allostérique ABL001, ou leurs sels.

10. Fibronectine à utiliser selon l'une quelconque des revendications précédentes, la fibronectine, ou un variant fonctionnel de celle-ci, ainsi que ses sels ou solvates, étant administrée à un patient atteint de leucémie après ou de manière concomitante à une transplantation de moelle osseuse.

11. Combinaison à utiliser dans le traitement de la LMC, la combinaison comprenant (a) de la fibronectine ou un variant fonctionnel de celle-ci, ainsi que ses sels ou solvates, et (b) l'inhibiteur de l'Abl1 ou un inhibiteur de l'expression, de la fonction et/ou la stabilité de la kinase liée à l'intégrine (ILK).

12. Combinaison à utiliser selon la revendication 11, l'inhibiteur de l'expression, de la fonction et/ou de la stabilité de la kinase liée à l'intégrine (ILK) étant (i) un anticorps inhibiteur de l'ILK, ou un fragment de liaison de l'antigène inhibiteur de celui-ci, ou (ii) une molécule nucléotidique antisens qui se lie à un acide nucléique qui code pour ILK, ou (iii) étant une molécule choisie parmi Cpd22, QLT0267, KP-392 ou T315, ou un isomère, un sel ou un solvate de ceux-ci.

13. Composition pharmaceutique à utiliser dans le traitement de la LMC, comprenant de la fibronectine ou un variant fonctionnel de celle-ci, ainsi que ses sels ou solvates, et un support, stabilisant et/ou excipient pharmaceutiquement acceptable.

14. Combinaison à utiliser selon l'une quelconque des revendications 11 à 12, la CML étant une CML positive pour Bcr-Abl1.

15. Combinaison à utiliser selon l'une quelconque des revendications 11, 12 ou 14, la CML étant est une CML résistante à l'imatinib, de préférence une CML positive mutante à Bcr-Abl1, telle que la CML positive à Bcr-Abl1^{T315I}.
